# EUROPEAN PATENT SPECIFICATION

(11) **EP 0 462 292 B1**
(45) Date of publication and mention of the grant of the patent: **03.01.1996**
(21) Application number: 91901904.2
(22) Date of filing: 09.01.1991
(51) Int. Cl.: A61K 31/725

(54) **SALT ELIMINANT COMPOSITION**
ZUSAMMENSETZUNG, WELCHE SALZ ENFERNT
PREPARATION ELIMINANT LE SEL

(30) Priority: 09.01.1990 JP 1003/90
(43) Date of publication of application: 27.12.1991
(73) Proprietor: KABUSHIKI KAISYA ADVANCE, Chuo-ku Tokyo 103 (JP)
(72) Inventor: ISHIHARA, Kazuoki, Kanagawa 229 (JP)
(74) Representative: Rinuy, Santarelli
(86) International application number: JP9100009
(87) International publication number: WO9110436

(56) References cited:
- JP-A-60 130 523
- PATENT ABSTRACTS OF JAPAN, vol. 8, no. 273 (C-256)(1710), 13 December 1984; & JP-A-59 143 559
- "Guide for Healthy Foods and Natural Foods, 1986", Jiyukokumin-sha, April 30, 1986, p. 166, paragraph of "vegetable fiber"

## Description

### TECHNICAL FIELD

The present invention relates to a novel sodium excretion-promoting composition. More particularly, the present invention relates to a composition which is useful in promoting the excretion of sodium ion contained in the cavity of the digestive tract by ingestion, which is in a dry state, is highly swellable in a salt solution such as a physiological saline, and is not digested in the digestive tract.

### BACKGROUND ART

Currently, it is considered that many food fibers which are not digestive promote the excretion of sodium ion, and serve to excrete toxic substances. These functions of food fibers are primarily attributed to (1) the ion exchange capacity thereof, and (2) the swellability (water retention) thereof. Many food fibers have an ion exchange capacity of about 0.1-0.4 meq/g (Nihon-Kaseigakukai-Si 39, 187-195(1988)), and thus, for an adsorption of sodium corresponding to 1 g of sodium chloride, it is necessary to ingest 40-200 g (dry weight) of food fibers; it is difficult, however, in everyday life, to ingest such an amount of food fibers. Regarding the swellability (water retention) thereof, agar can absorb and retain an amount of about four times the dry weight of a solution, alginic acid can absorb and retain an amount of about four to five times the dry weight of the solution, and wheat bran can absorb and retain an amount of about four times the dry weight of the solution. One gram of such food fibers can retain about 4 g of humor (liquor in the cavity of the digestive tract), which contains an amount of sodium corresponding to about 30 mg of sodium chloride. Therefore, to excrete 1 g of sodium chloride, it is necessary to ingest 30 to 40 g of food fibers, and although such an ingestion is not impossible, it is difficult to continue for a long time.

### DISCLOSURE OF THE INVENTION

Accordingly, the object of the present invention is to solve the problem of prior art as mentioned above and to provide a salt excretion-promoting composition having a high ion exchange capacity and swellability (water retention).

Other objects and features of the present invention will be apparent from the following description.

In accordance with the present invention, there is provided a salt excretion-promoting composition characterized by containing (i) 10 parts by weight of agar and (ii) 1 to 15 parts by weight of at least one polysaccharide selected from the group consisting of sodium alginate, λ- and κ-carrageenan and xanthane gum

### BEST MODE OF CARRYING OUT THE INVENTION

To attain the above-mentioned object, the present inventors made an intensive study, and consequently, found that the dry substance obtained by dissolving a mixture of the agar and the polysaccharide such as sodium alginate, λ- or κ-carrageenan or xanthane gum, in hot water, and solidifying (or gelling) the solution by cooling, followed by air-drying at an ordinary temperature, has a remarkably high swellability in a salt solution of such a physiological saline, and thus completed the present invention.

The ratio of agar to the other polysaccharide(s) in the mixture is preferably 1:0.1 to 1:1.5 (by weight), more preferably 1:0.5 to 1:1.0.

### EXAMPLES

The invention is illustrated by the following examples, and it is understood that the scope of the invention is not limited to these examples.

An amount of 1% of agar and various amounts (% by weight based on the weight of the aqueous solution) of sodium alginate, λ-carrageenan and κ-carrageenan were dissolved in hot water, and the solution was cooled and set (gelatinized). The obtained solid was then slowly air-dried at an ordinary temperature, and the swellability (the rate of the weight after swelling to the weight before swelling, in a physiological saline) of the thus-obtained dry substance was as shown in Table 1.

**Table 1**

| Additive other than agar (% by weight) | | Swellability |
|---|---|---|
| Sodium alginate | 1.0 | 32 |
| Sodium alginate | 0.5 | 25 |
| Sodium alginate | 0.2 | 18 |
| Sodium alginate | 0.1 | 12 |
| Gum arabic | 1 | 8.7 |
| λ-Carrageenan | 1 | 32 |
| κ-Carrageenan | 1 | 29 |
| Xanthane gum | 0.1 | 10 |
| Xanthane gum | 0.5 | 20 |
| Sodium alginate | 1.0 | |
| (Drying by heating at 60°C) | | 13 |
| (Drying by heating at 80°C) | | 7.5 |

The swellability was determined by leaving the dry substance to stand in a physiological saline (at 37°C, for 2 hours), taking out the formed gel, removing any solution adhering to the gel, measuring the total weight, and then dividing the total weight by the weight of the original dry substance.

### UTILIZABILITY IN INDUSTRY

As described above, according to the present invention, a remarkably swellable composition can be obtained, 3.3 to 4 g of the composition being satisfactory for the excretion of 1 g of sodium chloride, and the composition can be easily ingested in combination with other foods. Furthermore, the composition of the present invention can be used in the form of a powder, granule or the like, in combination with a salt solution such a physiological saline, or can be used in the form of the composition dispersed in a salt solution such as a physiological saline.

## Claims

1. A salt-excretion promoting composition, characterized by containing (i) 10 parts by weight of agar and (ii) 1 to 15 parts by weight of at least one polysaccharide selected from the group consisting of sodium alginate, λ- and κ-carrageenan and xanthane gum

2. A composition as claimed in claim 1, wherein said mixture of agar and polysaccaride is obtained by dissolving the mixture in water, gelatinizing the solution, and then air-drying the gel at an ordinary temperature.

## Patentansprüche

1. Salzausscheidung fördernde Zusammensetzung, dadurch gekennzeichnet, daß diese (i) 10 Gewichtsteile Agar und (ii) 1 bis 15 Gewichtsteile mindestens eines Polysaccharids aus der Gruppe bestehend aus Natriumalginat, λ- und κ-Carrageenan und Xanthan-Gummi enthält.

2. Zusammensetzung gemäß Anspruch 1, wobei die Mischung aus Agar und Polysaccharid durch Lösen der Mischung in Wasser, Gelatinierung der Lösung und Lufttrocknung des Gels bei normaler Temperatur erhalten wird.

## Revendications

1. Composition favorisant l'excrétion de sel, caractérisé en ce qu'elle contient (i) 10 parties en poids d'agar et (ii) 1 à 15 parties en poids d'au moins un polysaccharide choisissant le groupe consistant en alginate de sodium, carraghénine λ et κ et gommes xanthanes.

2. Composition suivant la revendication 1, dans laquelle le mélange d'agar et de polysaccharide est obtenu par dissolution de ce mélange dans l'eau, gélatinisation de la solution puis séchage du gel dans l'air à une température ordinaire.
